# EUROPEAN PATENT SPECIFICATION

(11) **EP 1 725 580 B1**
(45) Date of publication and mention of the grant of the patent: **28.01.2009**
(21) Application number: 05728233.7
(22) Date of filing: 17.03.2005
(51) Int. Cl.: C07K 1/107

(54) **ISOSTERIC TRANSFORMATION**
ISOSTERE TRANSFORMATION
TRANSFORMATION ISOSTERIQUE

(30) Priority: 17.03.2004 EP 04006354
(43) Date of publication of application: 29.11.2006
(73) Proprietor: AplaGen GmbH, 52499 Baesweiler (DE)
(72) Inventor: FRANK, Hans-Georg, NL-6462 EL Kerkrade (NL); KNORR, Karsten, 47807 Krefeld (DE); HABERL, Udo, 52499 Baesweiler (DE); RYBKA, Andreas, 45701 Herten (DE)
(74) Representative: Meyers, Hans-Wilhelm
(86) International application number: PCT/EP2005/051256
(87) International publication number: WO 2005/090389

(56) References cited:
- WO-A-01/53335
- WO-A-96/40749
- US-A- 4 638 046
- GROSS E & MEIENHOFER J (EDS.): "Peptides, Proceedings of the 6th American Peptide Symposium" 1979, PIERCE CHEM. CO. , ROCKFORD, ILL. (USA) , XP008057578 Chorev M et al.: Novel partially modified retro-inverso analogs of biologically active peptides, pages 455-458; figure 2 (peptide III), page 456
- WEINSTEIN B: "CHEMISTRY AND BIOCHEMISTRY OF AMINO ACIDS, PEPTIDES AND PROTEINS, PASSAGE" CHEMISTRY AND BIOCHEMISTRY OF AMINO ACIDS, PEPTIDES, AND PROTEINS, XX, XX, vol. 7, 1983, pages 266-357, XP002032461
- CHOREV M & GOODMAN M: "Recent developments in retro peptides and proteins - an ongoing topochemical exploration" TRENDS IN BIOTECHNOLOGY, ELSEVIER, AMSTERDAM, NL, vol. 13, no. 10, October 1995 (1995-10), pages 438-445, XP004207219 ISSN: 0167-7799
- SKELTON N J ET AL: "Amino acid determinants of beta-hairpin conformation in erythropoeitin receptor agonist peptides derived from a phage display library" JOURNAL OF MOLECULAR BIOLOGY, LONDON, GB, vol. 316, no. 5, 8 March 2002 (2002-03-08), pages 1111-1125, XP004472484 ISSN: 0022-2836
- RODERICK SL ET AL.: "Thiorphan and retro-Thiorphan Display Equivalent Interactions When Bound to Crystalline Thermolysin" BIOCHEMISTRY, vol. 28, 1989, pages 1493-1497, XP002308956
- GUICHARD G ET AL.: "Partially Modified Retro-Inverso Pseudopeptides as Non-natural Ligands for the Human Class I Histocompatibility Molecule HLA-A2" JOURNAL OF MEDICINAL CHEMISTRY, vol. 39, 1996, pages 2030-2039, XP002308957

## Description

### 1. Background of the Invention

The invention is in the field of the design and synthesis of retro-inverso peptides. Subject of the invention is an improved method for isosteric transformation, which is easier and faster to perform than conventional techniques. The invention also describes polypeptides obtainable by the method.

Numerous novel biotechnological drugs are proteins, which interact with receptors in the body. Examples of such successful drug developments are cytokines like Interleukin 2 and GM-CSF or G-CSF. Another important class of protein drugs are antibodies derived from totally recombinant systems such as Phage Display or from humanized mouse antibodies. Though being successful drugs, proteins as drugs have a number of disadvantages, which induce an ongoing search for what is called "small drugs" to substitute these biotechnological drugs on the long run. The most prominent disadvantages are short half life times in vivo due to proteolytic cleavage of the protein drugs and the high likelihood of immune responses to the protein drugs. Inactivating antibodies or allergic immune responses can even occur if minor contributions of incorrectly folded protein contaminate the protein preparation or if TAG's were used for purification or production purposes. Humanisation of antibodies - usually a time and cost intense procedure - can minimize these risks considerably, but can not abolish them.

However, the efforts to end up with classical pharmaceutical "small drugs" i.e. small organic molecules turned out to be a highly risky and so far not very successful approach. Very few examples, where a small drug could successfully target a complex and large protein receptor are known and could be established.

Among the valid alternatives peptides play a prominent role. Basically, they can be derived from complete proteins and can harbour the essential binding domains of larger proteins. They can be designed as both, agonists or antagonists of natural proteins on a given target. Their size usually allows chemical synthesis as an alternative to recombinant production. Moreover, small peptides are usually less immunogenic than large proteins. Small peptides might even be used in special galenic formulations for oral application. It has turned out that finding a suitable peptide for an intended pharmaceutical application is much easier and faster than finding an equivalent small drug.

Nevertheless, immunogenicity is not completely abolished and - more importantly - L-peptides are readily degradable in body fluids by numerous proteases and tend to have an even shorter half life than complete proteins. However, it is possible to use L-peptides derived from naturally occurring binding domains of proteins as a starting point to design non-biodegradable mimetics of L-peptides as the final drug candidates. Among the concepts, which are used for such purposes, the design of peptide isosters being constructed completely by non-natural, frequently by D-amino acids is very attractive. This is due to the fact that the same principles of synthesis and galenic preparation can be applied to such D-amino acid based peptides as to their L-amino acid based precursors. These mimetics of L-peptides are still peptides. Moreover, the risk of raising additional unexpected toxicity is for peptidic mimetics of L-peptides much smaller than for conventional organic small drugs.

An important approach to come up with a D-amino acid based L-peptidomimetic drug is the concept of retro-inverso peptides. These peptides are obtained by reversal of the amino acid sequence followed by inversion of the stereochemistry at the backbone alpha-carbon atoms (from L-amino acids to D-amino acids). While it is sometimes possible just to synthesize the retro-inverso peptide corresponding to a given L-peptide without considerable loss of biological activity, such a "trial and error" approach is highly risky and normally ends with failure. To minimize the risk of failure, a strategic planning of such a synthesis based on molecular modelling is needed in order to reveal basic structural problems associated with a given peptide and the intended stereochemical turnaround. Unfortunately, such molecular modelling of peptides is not trivial and requires a lot of time, efforts and skilled staff.

A large number of examples of both the success and failure of retro-inverso analogues of peptides have been reviewed in the literature [1, 2]. Recombinant techniques such as "mirror-image phage display" might also be used to obtain D-peptide based isosters of a given L-peptide, but are similarly risky and tideous as the actually available computer-based approaches:
So far, the standard procedures to design novel D-amino acid based isosteric peptides are time-consuming and do not automatically lead to conformations which mimic the receptor-bound active conformation of the native peptide. Generally, the state of the art computer-based procedure to generate a retro-inverso peptide as a conformational analogue to a given L-peptide comprises the following steps [3].

Initially, the sequence of the original peptide has to be reversed.

In a second step, the stereochemistry of the side chains at the alpha carbon atoms has to be changed from L to D.

To achieve conformational analogy between the original and the retro-inverso peptide, the backbone dihedral angle values phi and psi have to be interchanged for each corresponding residue.

While these three basic steps lead to just an intermediate structure additional transformations have to be performed, before the structure is ready for geometry optimization and molecular dynamics.

This complete procedure has three main disadvantages:
**Firstly,** it is complex, time-consuming and expensive. This is due to the fact, that steps 1 and 2 destroy the given structure of the original L-peptide completely. Sequence reversal and the change to D-amino acids reconfigure the arrangement of the side chains of the amino acids. These have to be reconstructed to a "near L-peptide" configuration in steps 3 and 4. Actually, this approach is just using the sequence of the L-peptide as a starting point. It does not make any use of the structural information (position and spatial orientation of side chains), which is available with the L-peptide. The whole process is thus an extremely time consuming "ab initio" modelling process.
**Secondly,** it can hardly be automatized or implemented in molecular modelling software.
**Thirdly,** the complete superimposition of the side chains with the native peptide is still not achievable. This problem will be even more difficult when a retro-inverso peptide has to be designed from a peptide-receptor complex to achieve a D-amino acid based isosteric peptide with receptor-bound conformation.

The main problem of any classical retro-inverso approach is that no structural information is preserved and a high risk of failure is associated with both, the "trial and error" synthesis and/or the "destructive/reconstructive" transformation of an L-peptide into a structurally analogous D-peptide.

The international patent publication WO 01/53335 discloses a method of generating retro-inverso peptides, wherein the candidate compound is selected from a database of three-dimensional structures of known compounds.

This invention overcomes these disadvantages of the state of the art by description of a simple procedure, which preserves the structural information associated with a given L-peptide. Furthermore, the invention offers solutions for typical structure- and sequence-related problems, which occur during an intended stereochemical turnaround of a given L-peptide precursor.

### 1. Description of the Invention

### 1.1. General Description

The present invention relates to isosteric transformation, a fast procedure enabling structure-based rational design of D-amino acid based peptides which act as isosters of corresponding native L-peptide precursors. In contrast to the conventional methods described above the invention can make use of structural information associated with a given L-peptide sequence (e.g. crystallographic data, NMR-data), usually showing this L-peptide being docked on its target binding site.

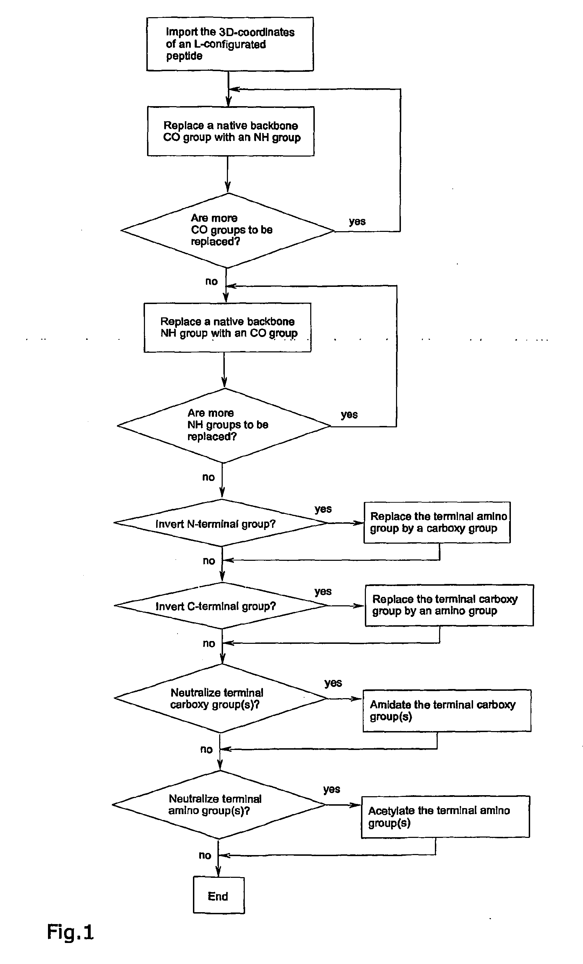

Subject of the invention are methods of any of claims 1 to 8. The central idea of the rational design according to the invention is to maintain the spatial orientation of the side chains of the amino acids during the whole procedure. Thus, there is no need for exchange of phi and psi dihedral angles and complete de novo construction of the intended molecule. The procedure according to the invention achieves this by just changing the backbone structure of the precursor peptide, while avoiding the exchange of complete amino acid moieties (no formal substitution of L-amino acids by D-amino acids). Since the peptide bonds of the backbone are strictly planar structures, this approach does not change the orientation or structural properties of the side chains, including that of covalent side chain modifications like disulfide bridges, helical constraints or other structural properties like e.g. helices or beta-sheets. The workflow of these operational steps can easily be automatized on standard modelling software. In particular, the method of the invention is performed according to the steps depicted in Figure 1. The method can be conducted on a computer device.

Isosteric transformation offers fast access to structural data of an intended D-analogue of an L-peptidic precursor. This allows instant assessment of the two main problems, which can render the newly designed molecule stereochemically incompatible with its precursor:
the occurrence of L-proline(s) in the sequence of the precursor, and the charges at the terminal ends of the peptide.

While synthetic solutions for modification of the charges at the ends of a peptide - if necessary - are available, the invention disclosed here offers a number of synthetic solutions for the proline problem, which can be used for structurally correct replacement of proline(s) during isosteric transformation. Isosteric transformation thus offers fast and attractive solutions for the stereochemical mirroring of L-peptide precursors - even of proline containing peptides - into respective D-peptide analogues.

The invention also relates to the synthesis of these newly designed compounds and relates to peptides containing building blocks and terminal modifications in D-peptides according to the invention. In general, the invention provides solutions which can be adapted to almost every peptidic structure provided sufficient information on the L-peptidic structure serving as the starting point was available.

It is clear to persons skilled in the art, that the principle of the invention disclosed herein, can be used also without the availability of detailed structural data, e.g. based on sequence information alone. However, this implies that a "trial and error" approach in the synthesis laboratory has to be used in connection with suitable e.g. in vitro test systems to identify active and structurally valid D-amino acid based substances. Though making use of the principle of this invention, this approach does not share all advantages - especially the fast lead-finding opportunity - with the complete structure-based implementation of the invention as disclosed below. It is clear, however, that such partial implementations of the inventive principle are also covered by the procedure of this invention.

### 1.2. Detailed Description

The present invention discloses a method for designing a peptide isoster or peptide-like substance based on the coordinates of the structure of a native peptide by the inversion of one ore more (up to all) peptide bonds, comprising steps a-c:
Importing the structural data of the given L-peptide into a suitable computer device, on which a software is loaded, which is able to handle structural data of the given peptide conformation
replacing the atoms of the native carbonyl-(CO) backbone-group by an amide-(NH) group,
replacing the atoms of the native amide-(NH) backbone-group by a carbonyl-(CO) group.
Steps a to c can be performed manually by a skilled staff member using the given software utility or can be automatized by appropriate programming of the computer/software unit. Even for a complex peptidic structure, steps a to c can be passed within a few hours. These steps will end in an intermediate structure, which contains all side chains in correct spatial orientation and shows an already inverted backbone relating to a D-peptide structure. The terminal part of the peptide is not yet inverted. Thus, this primary product structure has a non-terminally inverted backbone (see example below, reduced to a two-dimensional sketch).

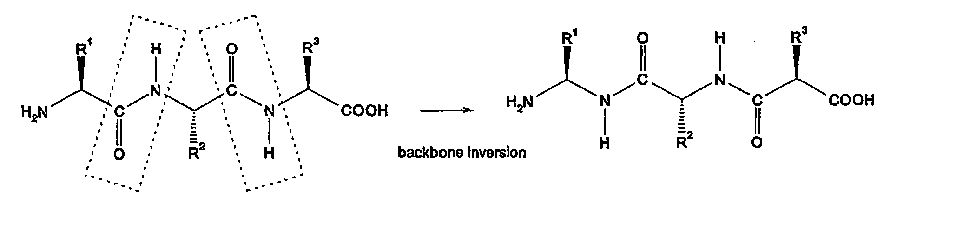

In a further aspect of the invention, the C-terminal and/or N-terminal end groups can be modified in addition to non-terminal backbone inversion. The C-terminal carboxyl group can be interchanged by an amino group and/or the N-terminal amino group can be interchanged by a carboxyl group:

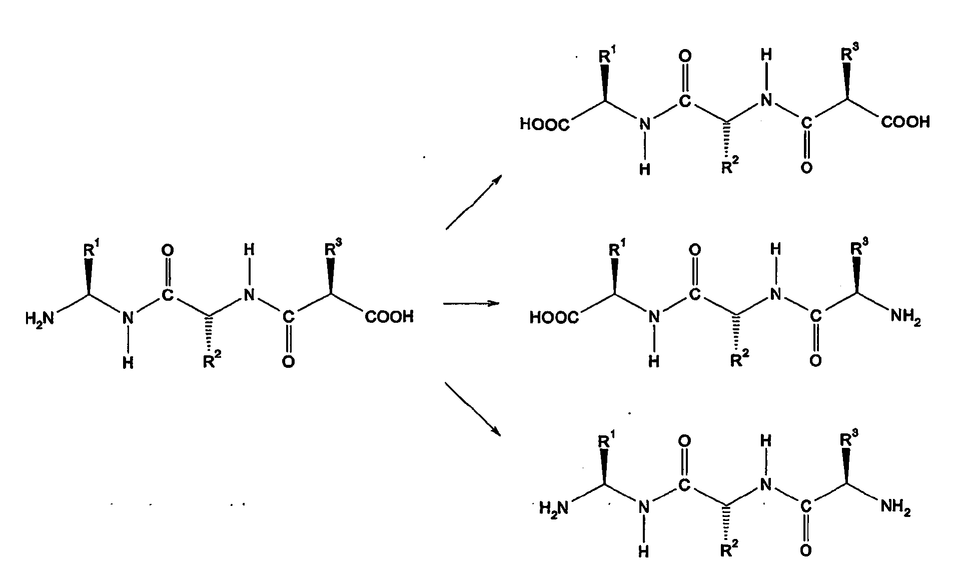

These additional Steps lead to 3 additional molecules, which together with the primary product structure serve as the starting point for optimisation of the overall design according to the invention (see below). In some of these cases, gem-diaminoalkyl and C-2-substituted malonyl residues are incorporated as terminal end groups. The synthesis of such building blocks has been reviewed in detail. [4]

So far, this method has already three main advantages as compared to the classical methods of isoster design:
It is easy and quickly to perform.

It can easily be automatized or implemented in molecular modelling programs. The geometry and conformation of the side chains of the resulting structure, which is the starting point for further optimization and dynamics, does not change in comparison to the native peptide. This is a big benefit, especially for the design of receptor-bound peptides, because this method generates isosters which maintain the same side chain conformation as the native peptide. Thus, the resulting primary product structure is an isoster and has equivalent sidechain interactions with the receptor protein as the original L-peptide.

The following two-dimensional figure illustrates these advantages of the isosteric transformation method: it takes fewer operations and the geometry of the side chains is never changed.

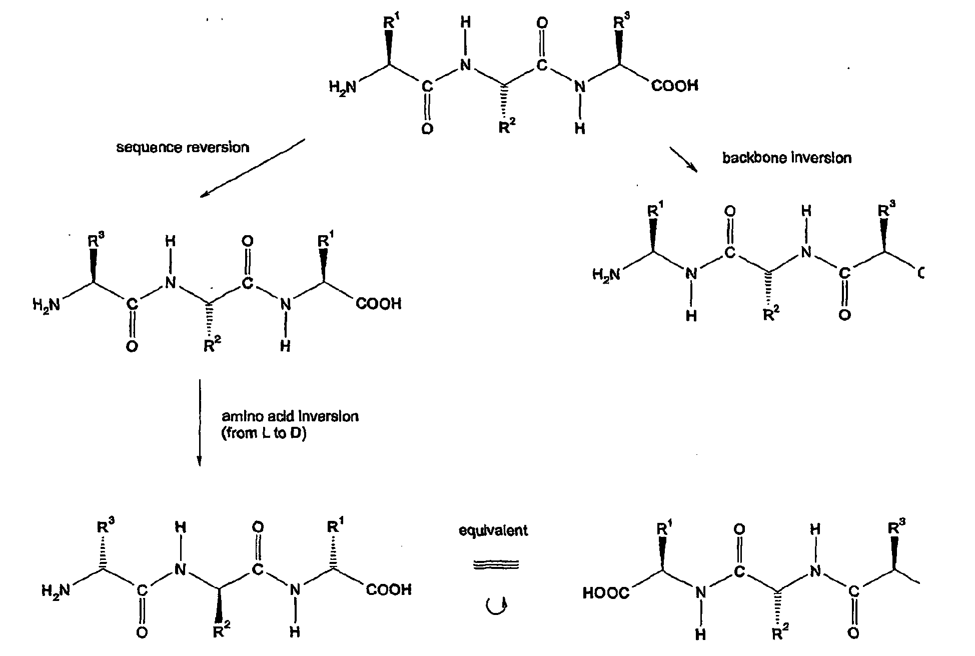

This invention also describes polypeptides based on D-amino acids obtained by the method described above. Due to the fact that this method is a general way to generate isosteric structures of L-peptides, the invention also describes compounds which are isosteric to proline-containing peptides.

Thus, the present invention also describes building blocks which are important when the native peptide contains one ore more proline residues. Proline is often considered incompatible with the conventional retro-inverso approach [5], even if examples of proline-containing retro-inverso peptides retain biological activity [6]. Proline is the only natural cyclic amino acid in which the side chain is tethered to the alpha amino group and thus back to the backbone. This property defines a special for isosteric transformation, which is based on backbone inversion and thus leads in the case of proline - as an exception among the amino acids - to stereochemical distortion during isosteric transformation. Moreover, this constraint dictates restricted backbone dihedral angles that are different to those found in peptides not involving proline. In standard retro-inverso peptides, directional reversion of proline leads to topochemical displacement of the pyrrolidine ring in comparison to the native peptide. This is also true for the isosteric transformation method presented here. However, by isosteric transformation as described above, the non-terminally backbone-inverted peptide can be tested e.g. by molecular dynamics for possible incompatibility with the structural requirements defined by the original L-peptidic structure. Thus, already before synthesis and in vitro testing, a rationale can be developed, whether the proline problem is actually an important aspect of the given structure. In case that reversal of the backbone distorts the structure at the proline position unacceptably, the isosteric transformation method offers a number of solutions for this specific but frequent problem:
In structures like beta-turns or beta-hairpins, proline can often be replaced by a glycine after isosteric transformation:

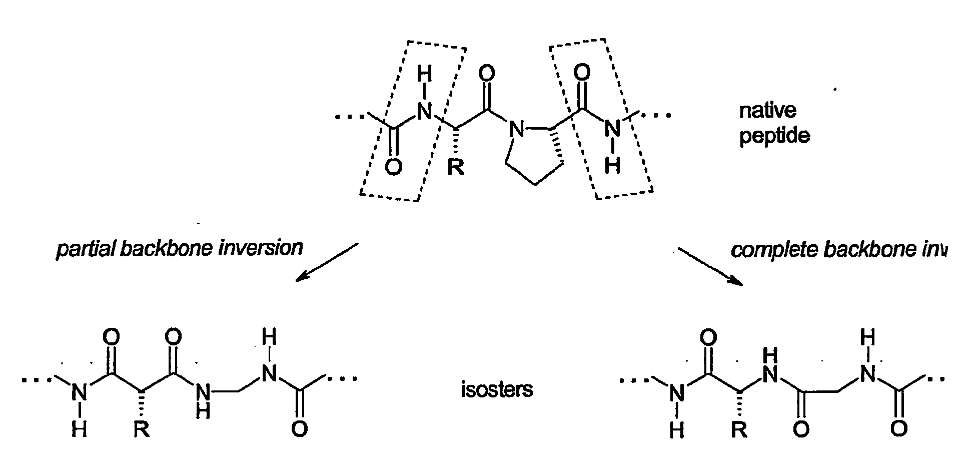

Further described are, in addition to isosteric transformation, two amino acid units (the proline and the immediately neighbouring residue) can be replaced by one building block like 5-Aminovaleric acid or its derivatives:

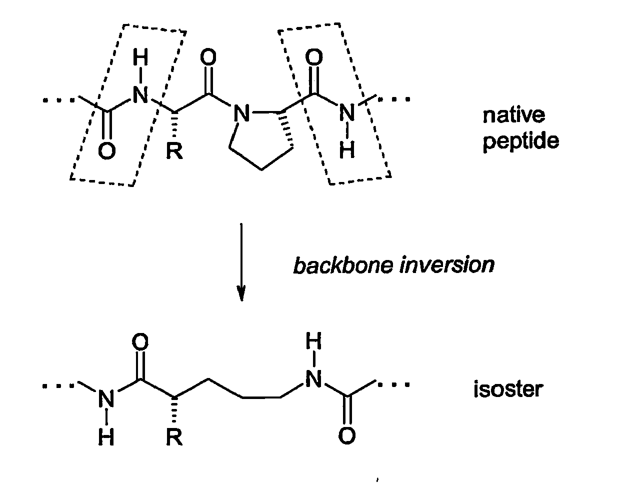

Generally, after isosteric transformation, two amino acids (the proline and the immediately neighbouring residue) can be replaced by a building block represented by the generic formula

...-(CO)-x¹-x²-X³-X⁴ -NH-...

Wherein X¹, X², X³, and X⁴ are independently selected from CH₂, (C=O), NH, NR, O, (CHR), or (CR₂), wherein R in an amino group, an alcohol, halogen or any organic residue. The following figure shows some examples of the use of building blocks described by this general formula:

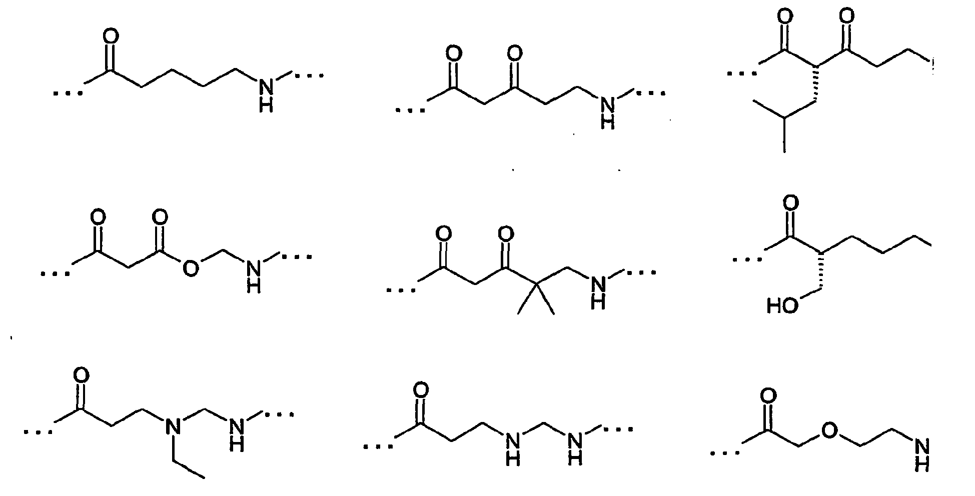

The invention also describes cyclic building blocks which mimic the conformation of the proline residue after isosteric transformation and replace proline and its neighbouring amino acid residue:

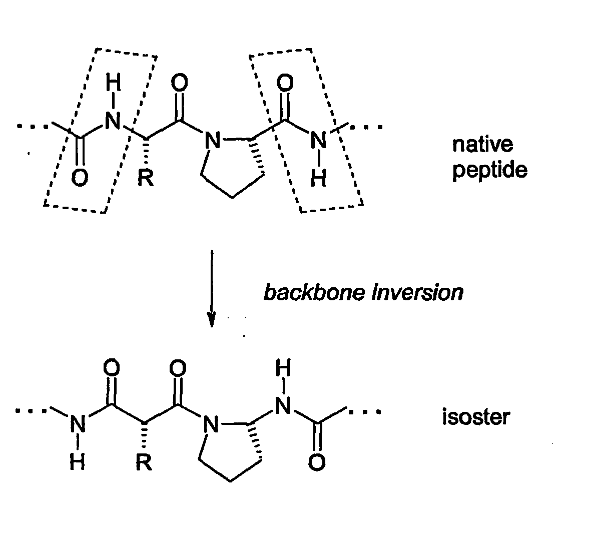

The use of such a building block is useful and possibly necessary in cases where glycine or extended glycine equivalent structures like 5-aminovaleric acid derivatives do not suffice to stabilize the sterical properties of a given peptide adequately. This is especially true, where an "induced fit" into the target structure becomes problematic for the peptide without adequate reconstruction of proline-specific conformations. Proline-replacing building blocks like the one shown here for replacement of proline and a neighbouring glycine can be constructed according to the generic formula given below:
Further described are, after isosteric transformation, two neighbouring amino acids (one of which was proline) can be replaced by a building block represented by the generic formula

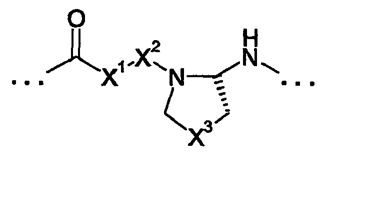

Wherein X¹, X² and X³ are independently selected from CH₂, (C=O), O, S, NH, NR, (CHR), or (CR₂), wherein R in an amino group, an alcohol, halogen or any organic residue. The following figure shows some examples of building blocks described by this general formula:

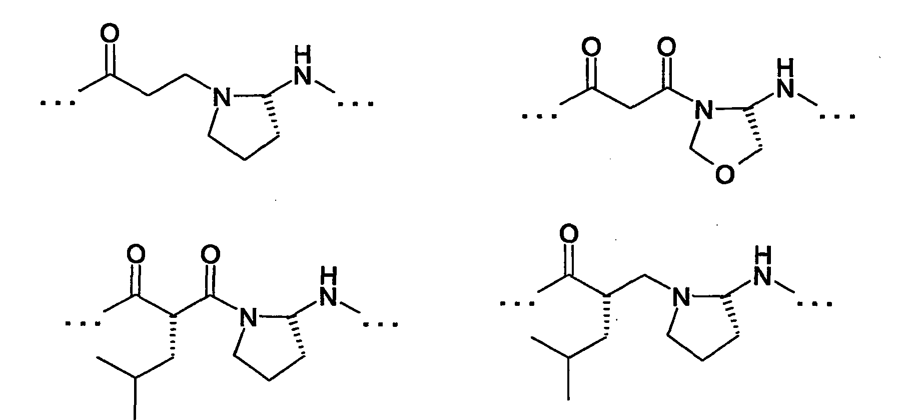

The use of such a building block is useful and possibly necessary in cases where glycine or extended glycine equivalent structures like 5-aminovaleric acid derivatives do not suffice to stabilize the sterical properties of a given peptide adequately. This is especially true, where an "induced fit" into the target structure becomes problematic for the peptide without adequate reconstruction of proline-specific conformations.

Further disclosed are, after isosteric transformation, two amino acids can be replaced by a building block represented by the generic formula 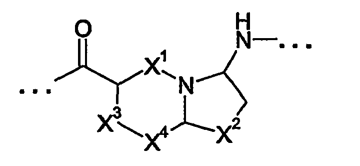 wherein X¹, X², X³ and X⁴ are independently selected from CH₂, (C=O), O, S, NH, NR, (CHR), or (CR₂), wherein R in an amino group, an alcohol, halogen or any organic residue. The following figure shows some examples of building blocks described by this general formula:

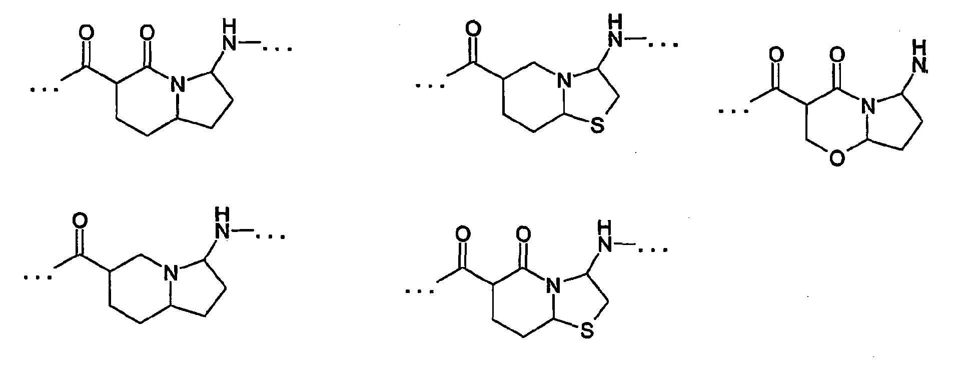

The use of such a building block is useful and possibly necessary in cases where glycine or extended glycine equivalent structures like 5-aminovaleric acid derivatives do not suffice to stabilize the sterical properties of a given peptide adequately. This is especially true, where an "induced fit" into the target structure becomes problematic for the peptide without adequate reconstruction of proline-specific conformations. The molecules based on the generic formula shown here can be used alternatively to the pyrrolidin-based structures shown earlier.

The building blocks and the use of the building blocks as outlined above are especially useful in isosteric transformation of polypeptides comprising proline according to the invention. However, they are also useful in conventional techniques for the design and production of D-peptides and retro-inverso peptides.

Amino acids described in this invention can be of the naturally occurring L-stereoisomer form as well as the enantiomeric D form. The one-letter code refers to the accepted standard polypeptide nomenclature, but can mean alternatively a D- or L-amino acid. Lower case letters refer to D amino acids:
- A: L-Alanine or D-Alanine
- V: L-Valine or D-Valine
- L: L-Leucine or D-Leucine
- I: L-Isoleucine or D-Isoleucine or D-*allo*-Isoleucine
- M: L-Methionine or D-Methionine
- F: L-Phenylaianine or D-Phenylalanine
- Y: L-Tyrosine or D-Tyrosine
- W: L-Tryptophan or D-Tryptophan
- H: L-Histidine or D-Histidine
- S: L-Serine or D-Serine
- T: L-Threonine or D-Threonine or D-*allo*-Threonine
- C: L-Cysteine or D-Cysteine
- N: L-Asparagine or D-Asparagine
- Q: L-Glutamine or D-Glutamine
- D: L-Aspartic acid or D-Aspartic acid
- E: L-Glutamic acid or D-Glutamic acid
- K: L-Lysine or D-Lysine
- R: L-Arginine or D-Arginine
- P: L-Proline or D-Proline
- G: Glycine
- a: D-Alanine
- v: D-Valine
- I: D-Leucine
- i: D-Isoleucine or D-*allo-*Isoleucine
- m: D-Methionine
- f: D-Phenylalanine
- y: D-Tyrosine
- w: D-Tryptophan
- h: D-Histidine
- s: D-Serine
- t: D-Threonine or D-*allo-*Threonine
- c: D-Cysteine
- n: D-Asparagine
- q: D-Glutamine
- d: D-Aspartic acid
- e: D-Glutamic acid
- k: D-Lysine
- r: D-Arginine
- p: D-Proline

### 2. Examples

### 2.1. Isosteric transformation of peptides

### 2.1.1 Isosteric transformation of a proline-free helical peptide which binds to the beta chain of the interleukin-2 receptor

The following example demonstrates the transformation of a helical peptide which binds to the beta chain of the interleukin-2 receptor.

This peptide - in analogy to interleukin-2 (IL-2) - has the capability to bind to IL-2 specific receptors with high affinity. IL-2 is a cytokine used in tumor therapy. IL-2 binds to specific receptors (IL-2R) whereby the IL-2-specific intracellular signals are triggered.

IL-2 has stimulating effects on the growth of T and B lymphocytes, activates cytotoxic and cytolytic NK cells. Thus it has a central significance in the regulation of the immune response. Thus, IL-2 is of fundamental importance in the immune response to tumors and inflammatory reactions. One of the mechanisms which is of importance for the tumor defense with IL-2 seems to be the induction of LAKs ("lymphokine activated killer cells"). These cells are able to destroy tumor cells.

The structure file with the coordinates of the native peptide contains the structure of the complex between a helical peptide with the formula **STKKTQLQLEHLLLDLQMILNGINNY** and the beta and gamma chain of the interleukin-2 receptor. This helical peptide offers an ideal example for the implementation of isosteric transformation:
The coordinates of the homodimeric peptide in receptor-bound conformation are imported into molecular modelling software.
The atoms of the native carbonyl-(CO) backbone groups are replaced by amide-(NH) groups.
The atoms of the native amide-(NH) backbone groups are replaced by carbonyl-(CO) groups.

The procedure of isosteric transformation leaves the conformation of the side chain atoms unchanged, because the modifications only take place in the backbone. Therefore this method is a good procedure to generate a starting conformation for the design and optimization of a receptor-binding peptide which is based on D-amino acids.

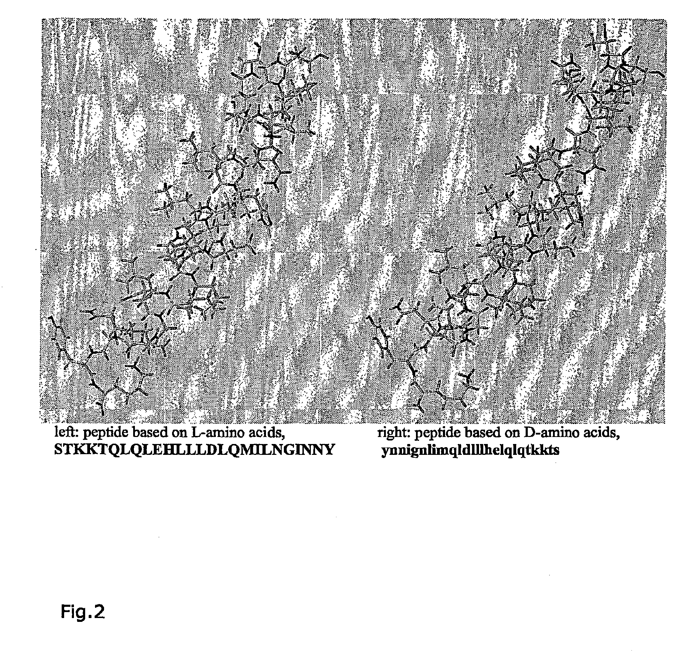

A comparison of the structure of the native helical peptide with the resulting structure after isosteric transformation and geometry-optimization demonstrates the structural equivalence of the native peptide and the transformed molecule is shown in figure 2.

One of the effects of the backbone reversion is not only the reversion of the amino acid sequence but also the inversion of the configuration of the alpha carbon atoms of the amino acids, which changes from L to D. This is indicated by lower case letters in the amino acid sequences in this invention. Threonine contains two chiral centres and therefore, while maintaining side-chain chirality, the appropriate isoster is D-*allo*-Thr, which is indicated by a lower case "t" in the peptide sequence formulas.

Overall, the isosteric transformation of the L-peptide with the formula **STKKTQLQLEHLLLDLQMILNGINNY** is transformed to a D-peptide which can be described with the formula **ynnignlimqldlllhelqlqtkkts**. The present invention discloses a fast method to generate coordinates of an isosteric, receptor-docked structure of this peptide.

The resulting peptide is entirely composed of D-amino acids. This leads to longer biological half-life in comparison with the native L-peptide. A peptide based on D-amino acids is more stable according to proteolytic enzymes. Thus, the isosterically tranformed peptide mimetic is designed to have better properties as a pharmaceutical than the L-peptide.

The D-peptide is suited for the treatment of diseases of the immunologic system, e.g., inflammations and arthritic processes or of immunodeficiency syndromes of all types and genesis; diseases connected with an increased proliferation of cells, e.g., carcinoses, for example in the form of carcinomas, sarcomas, lymphomas and leukaemias; or infectious processes.

### 2.1.2 Isosteric transformation of a peptide which binds to the erythropoietin receptor

The following example demonstrates the transformation of a peptide which binds to the erythropoietin receptor.

The coordinates which represent the native structure are publicly available in the Protein Data Bank (PDB code: IEBP). This file contains the structure of the complex between a dimeric agonist peptide with the formula **TYSCHFGPLTWVCKPQ** and the dimeric erythropoietin receptor [7]. This structurally well-documented peptide offers an ideal example for the implementation of isosteric transformation:
The coordinates of the homodimeric peptide in receptor-bound conformation are imported into appropriate modules of the Sybyl modelling software.
The atoms of the native carbonyl-(CO) backbone groups are replaced by amide-(NH) groups.
The atoms of the native amide-(NH) backbone groups are replaced by carbonyl-(CO) groups.

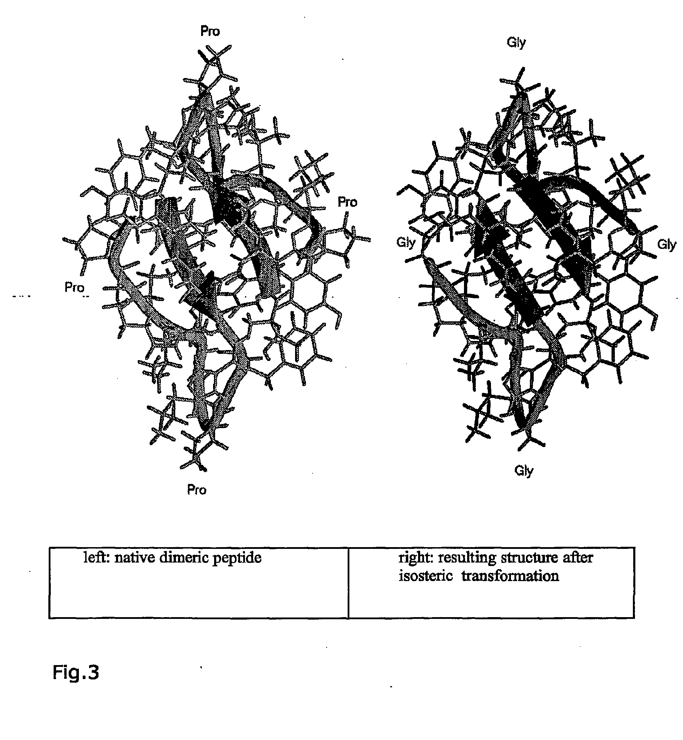

Since the peptide sequence harbours two proline residues, structural distortions at the site of the prolines occur in the primary structure obtained by isosteric transformation. However, it is also obvious, that the secondary structure of the peptide in "induced fit" position is stabilized by numerous other molecular interactions and does potentially not require the specific conformations induced by proline-specific properties. Proline - though supportive for the induced fit conformation - is thus not essential for it and can be replaced in the most simple case by the very flexible glycine. The procedure of isosteric transformation leaves the conformation of the side chain atoms (with the exception of the native proline residues) unchanged, because the modifications only take place in the backbone. A comparison of the structure of the native dimeric peptide with the resulting structure after isosteric transformation and replacement of prolines by glycines demonstrates the structural equivalence of the native peptide and the transformed molecule is shown in figure 3.

The direction of the arrows in figure 3 indicates the direction of the backbone from the N-terminal ends to the C-terminal ends. After isosteric transformation, the structure is ready for further geometry optimization and molecular dynamics simulations.

One of the effects of the backbone reversion is not only the reversion of the amino acid sequence but also the inversion of the configuration of the alpha carbon atoms of the amino acids, which changes from L to D. This is indicated by lower case letters in the amino acid sequences in this invention. Threonine contains two chiral centres and therefore, while maintaining side-chain chirality, the appropriate isoster is D-*allo*-Thr, which is indicated by a lower case "t" in the peptide sequence formulas.

The resulting molecule structure can be described by the following formula:

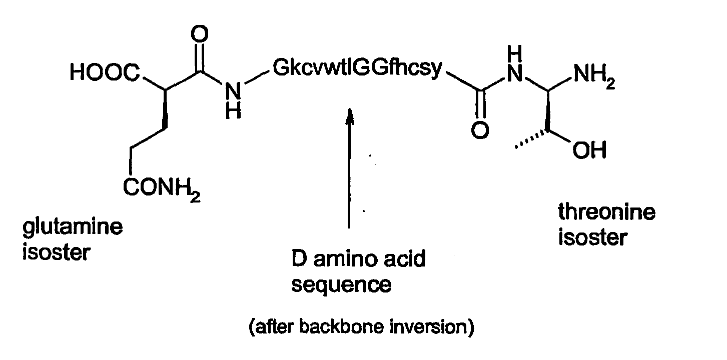

In addition to the steps above, the C-terminal carboxyl groups are interchanged by amino groups and the N-terminal amino groups are interchanged by carboxyl groups.

Overall, the isosteric transformation of an L-peptide with the formula **TYSCHFGPLTWVCKPQ** is transformed to a D-peptide which can be described with the formula **qGkcvwtlGGfhcsyt**.

This formula includes possible modifications of the C- and N-terminal ends of the peptide by e.g. addition of additional non-binding amino acids such as glycine or alanine as well as amidation and/or acetylation of the N- and C-terminal ends.

### 2.1.3 Substitution of two amino acids by 5-aminovaleric acid

As a further example of an isosteric transformation of the native peptide shown in 2.1.2, two amino acids are replaced by 5-aminovaleric acid (5-Ava) additionally to the operations described in 2.1.2. Overall, the isosteric transformation of an L-peptide with the formula **TYSCHPGPLTWVCKPQ** is transformed to a D-peptide which can be described with the formula **qGkcvwtl-(5-Ava)-fhcsyt.**

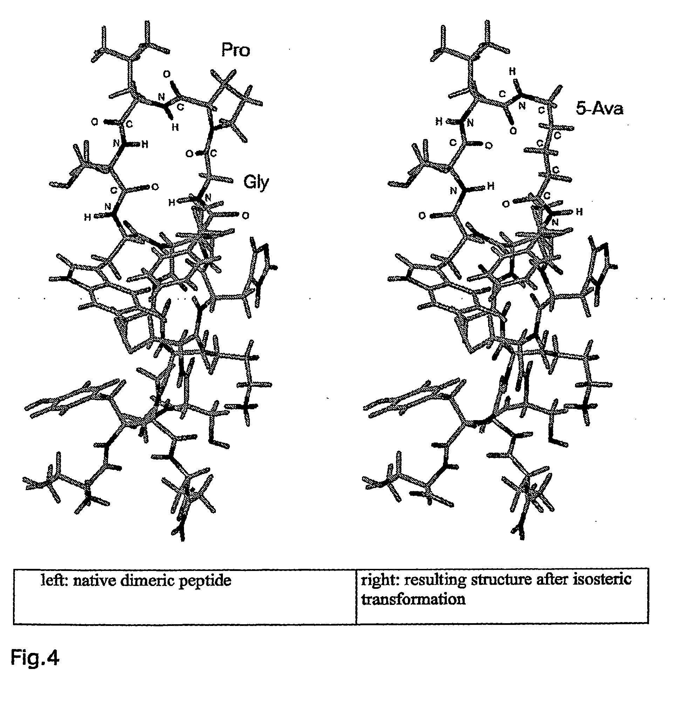

The use of this building block is illustrated by the structures shown in figure 4.

### 2.2. Synthesis of the building block 3-(2S-Allyloxycarbonylaminopyrrolidin-1-yl)-3-oxo-propionic acid:

In addition to replacement of proline by structurally inert or just flexible residues like glycine it might be necessary or advantageous to reconstruct the properties, which were induced by L-proline in the precursor peptide by an appropriate synthetic building block mimicking a retro-proline in the peptide obtained by isosteric transformation. Below we describe the synthesis of a molecule, which can fulfil this type of building block function in e.g. the abovementioned erythropoietin-mimetic peptide by replacing proline and its neighbouring glycine residue:

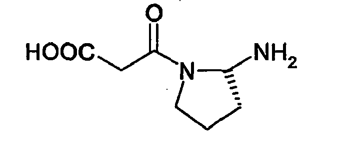

### Precursor 1-(2-Methoxycarbonyl-acetyl)-pyrrolidine-2S-carboxylic acid:

L-Proline (3.45 g, 30 mmol) was dissolved in 30 ml 1N NaOH und diluted with 30 ml water. Stirring vigourously methyl 3-chloro-3-oxopropionate (4.10 g, 30 mmol) and 30 ml 1N NaOH were dropped simultanously at 0°C. The solution was stirred for one hour at 0°C and another hour at room temperature. The mixture was acidified with saturated NaHSO₄ solution to pH = 1-2 and extracted several times with ethyl actetate. The combined organic layers were dried (Na₂SO₄), the solvent removed *in vacuo,* and the resulting crude oil purified by silica column chromatography.

### Precursor: 3-(2S-Allyloxycarbonylamino-pyrrolidin-1-yl)-3-oxo-propionic acid methyl ester:

Diisopropylethylamine (0.97 g, 7.5 mmol) was added dropwise to a solution of 1-(2-Methoxycarbonyl-acetyl)-pyrrolidine-2S-carboxylic acid (1.08 g, 5.0 mmol) in acetone (15 ml) and water (0.5 ml) at 0°C. Ethyl chloroformate (0.76 g, 7.0 mmol) was added dropwise and the solution stirred for 30 min at 0°C. 4 N aqueous NaN₃ (2.0 ml, 8.0 mmol) was added and the solution stirred for additional 3 h at 0°C. The solution was partitioned between H₂O (20 ml) and Et₂O (30 ml) and the aqueous layer extracted with Et₂O (4 x 30 ml). The combined organic layers were dried (MgSO₄) and toluene (20 ml) added. Et₂O was removed under reduce pressure, and allyl alcohol (1.37 ml, 20 mmol) added. The reaction mixture was heated at reflux for 4 h. The solvent was removed under reduced pressure and the resulting oil purified by silica column chromatography.

### 3-(2S-Allyloxycarbonylamino-pyrrolidin-1-yl)-3-oxo-propionic acid:

3-(2S-Allyloxycarbonylamino-pyrrolidin-1-yl)-3-oxo-propionic acid methyl ester (1.35 g, 5.0 mmol) was dissolved in 2-hexanone (6 ml). Dry lithium bromide (0.48g, 5.5 mmol) was added, and the reaction mixture heated at reflux for 1 h. The cooled mixture was partitioned between water (20 ml) and Et₂O (30 ml). The aqueous layer was extracted with Et₂O (3 x 30 ml), the combined organic layers dried (MgSO₄), and the solvent removed in vacuo. The resulting solid was recrystallized from ethyl acetate/hexane.

### References:

1. MHVv Regenmortel, S Muller: D-peptides as immunogens and diagnostic reagents. Current Opinion in Biotechnology 1998, 9:377-382.
2. PM Fischer: The Design, Synthesis and Application of Stereochemical and Directional Peptide Isomers: A Critical Review. Current Protein and Peptide Science 2003, 4:339-356.
3. DT Nair, KJ Kaur, K Singh, P Mukherjee, D Rajagopa, A George, V Bal, S Rath, KVS Rao, DM Salunke: Mimicry of Native Peptide Antigens by the Corresponding Retro-Inverso Analogs Is Dependent on Their Intrinsic Structure and Interaction Propensities 1. The Journal of Immunology 2003, 170:1362-1373.
4. MD Fletcher, MM Campbell: Partially Modified Retro-Inverse Peptides: Development, Synthesis, and Conformational Behavior. Chemical Reviews 1998, 98:763-796.
5. K Vogler, P Lanz, W Lergier, W Haefely: [Synthesis of bradykinin analogs with D-amino acids (all-D-bradykinin and all-D-retro-bradykinin)]. Helv Chim Acta 1966, 49:390-403.
6. T Wieland, B Penke, C Birr: [Antamanide. XVI. All-D-retro-antamanide and D-tyr 6 -all-D-retro-antamanide]. Justus Liebigs Ann Chem 1972, 759:71-5.
7. O Livnah, EA Stura, DL Johnson, SA Middleton, LS Mulcahy, NC Wrighton, WJ Dower, LK Jolliffe, IA Wilson: Functional mimicry of a protein hormone by a peptide agonist: the EPO receptor complex at 2.8 A. Science 1996, 273:464-471.

## Claims

1. A method of generating an isosteric structure of a polypeptide at least partially containing D-amino acids from 3D-coordinates and sequence information of an L-configurated precursor having an N-terminal amino group or substituted amino group, a C-terminal carboxy group or a carboxy derivative, a backbone and L-amino acid side chains, and comprising the steps of
- at least partially replacing backbone CO groups with NH groups and vice versa,
- while keeping fixed the 3D-coordinates of the precursors L-amino acid side chains, the N-terminal amino group or substituted amino group and the C-terminal carboxy group or carboxy derivative.

2. The method according to claim 1 comprising the steps of
- at least partially replacing backbone CO groups with NH groups and vice versa,
- while keeping the 3D-coordinates of the precursor's L-amino acid side chains fixed, and replacing the N-terminal amino group or substituted amino group by a carboxy group or carboxy derivative and/or replacing the C-terminal carboxy group or carboxy derivative by an amino group or substituted amino group.

3. The method according to claims 1-2 wherein all backbone CO groups of the precursor are replaced by NH groups and vice versa.

4. The method according to claims 1-3, **characterized by**
- at least partially replacing the proline residues or proline residues and their adjacent neighbouring residue in the structure and sequence of the precursor by organic molecules as building blocks mimicking the conformational properties of proline or of proline and its immediately neighbouring residue in the newly configured backbone.

5. The method according to claims 1 to 4 comprising the steps according to figure 1.

6. The method according to claims 1-5 conducted on a computer device.

7. A method of generating a polypeptide comprising at least one D-amino acid, the method comprising the steps of obtaining an isosteric structure by a method of any of claims 1 to 6 and synthesizing the polypeptide of said isosteric structure.

8. The method of claim 7, wherein the polypeptide consists of D-amino acids.

## Patentansprüche

1. Verfahren zur Erzeugung einer isosterischen Struktur eines Polypeptids, das wenigstens teilweise D-Aminosäuren enthält, aus 3D-Koordinaten und Sequenzinformationen eines L-konfigurierten Vorläufers mit einer N-terminalen Aminogruppe oder substituierten Aminogruppe, einer C-terminalen Carboxygruppe oder einem Carboxyderivat, einem Gerüst und L-Aminosäureseitenketten, umfassend die Schritte:
- wenigstens partielles Ersetzen von Gerüst-CO-Gruppen durch NH-Gruppen und umgekehrt;
- während die 3D-Koordinaten der L-Aminosäureseitenketten, der N-terminalen Aminogruppe oder substituierten Aminogruppe und der C-terminalen Carboxygruppe oder des Carboxyderivats des Vorläufers festgehalten werden.

2. Verfahren gemäß Anspruch 1, umfassend die Schritte:
- wenigstens partielles Ersetzen von Gerüst-CO-Gruppen durch NH-Gruppen und umgekehrt;
- während die 3D-Koordinaten der L-Aminosäureseitenketten des Vorläufers festgehalten werden und die N-terminale Aminogruppe oder substituierte Aminogruppe durch eine Carboxygruppe oder ein Carboxyderivat ersetzt wird und/oder die C-terminale Carboxygruppe oder das Carboxyderivat durch eine Aminogruppe oder substituierte Aminogruppe ersetzt wird.

3. Verfahren gemäß Anspruch 1 bis 2, wobei alle Gerüst-CO-Gruppen des Vorläufers durch NH-Gruppen ersetzt werden und umgekehrt.

4. Verfahren gemäß Anspruch 1 bis 3, **gekennzeichnet durch**:
- wenigstens partielles Ersetzen der Prolinreste oder von Prolinresten und ihres direkt benachbarten Rests in der Struktur und Sequenz des Vorläufers **durch** organische Moleküle als Bausteine, die die konformatorischen Eigenschaften von Prolin oder von Prolin und seines direkt benachbarten Rests in dem neu konfigurierten Gerüst nachbilden.

5. Verfahren gemäß Anspruch 1 bis 4, das die Schritte gemäß Figur 1 umfasst.

6. Verfahren gemäß Anspruch 1 bis 5, das an einer Computervorrichtung durchgeführt wird.

7. Verfahren zur Erzeugung eines Polypeptids, das wenigstens eine D-Aminosäure umfasst, wobei das Verfahren die Schritte des Gewinnens einer isosteren Struktur nach einem Verfahren gemäß einem der Ansprüche 1 bis 6 und des Synthetisierens des Polypeptids der isosteren Struktur umfasst.

8. Verfahren gemäß Anspruch 7, wobei das Polypeptid aus D-Aminosäuren besteht.

## Revendications

1. Procédé de production d'une structure isostérique d'un polypeptide contenant au moins en partie des acides aminés D à partir de coordonnées 3D et d'informations de séquence d'un précurseur configuré en L ayant un groupe amino N-terminal ou un groupe amino substitué, un groupe carboxyle C-terminal ou un dérivé de carboxyle, une chaîne principale et des chaînes latérales d'acides aminés L, et comprenant les étapes consistant à :
- remplacer au moins en partie les groupes CO de la chaîne principale par des groupes NH et *vice versa,*
- tout en gardant fixes les coordonnées 3D des chaînes latérales d'acides aminés L des précurseurs, du groupe amino N-terminal ou du groupe amino substitué et du groupe carboxyle C-terminal ou du dérivé carboxyle.

2. Procédé selon la revendication 1, comprenant les étapes consistant à :
- remplacer au moins en partie les groupes CO de la chaîne principale par des groupes NH et *vice versa,*
- tout en gardant fixes les coordonnées 3D des chaînes latérales d'acides aminés L des précurseurs, et en remplaçant le groupe amino N-terminal ou le groupe amino substitué par un groupe carboxyle ou dérivé carboxyle et/ou en remplaçant le groupe carboxyle C-terminal ou le dérivé carboxyle par un groupe amino ou un groupe amino substitué.

3. Procédé selon les revendications 1 à 2, dans lequel tous les groupes CO de la chaîne principale du précurseur sont remplacés par des groupes NH et *vice versa.*

4. Procédé selon les revendications 1 à 3, **caractérisé par** :
- le remplacement au moins partiel des groupes proline ou des groupes proline et de leur groupe adjacent dans la structure et la séquence du précurseur par des molécules organiques tels que des briques de construction imitant les propriétés conformationnelles des groupes proline ou des groupes proline et de leur groupe adjacent dans la chaîne principale nouvellement conçue.

5. Procédé selon les revendications 1 à 4, comprenant les étapes selon la figure 1.

6. Procédé selon les revendications 1 à 5, réalisé sur un dispositif d'ordinateur.

7. Procédé de production d'un polypeptide comprenant au moins un acide aminé D, le procédé comprenant les étapes consistant à obtenir une structure isostérique par un procédé selon l'une quelconque des revendications 1 à 6, et à synthétiser le polypeptide de ladite structure isostérique.

8. Procédé selon la revendication 7, dans lequel le polypeptide se compose d'acides aminés D.
